Europäisches Patentamt

European Patent Office

Office européen des brevets

(19)

(11) Publication number: **0 115 781**
**B1**

# EUROPEAN PATENT SPECIFICATION

(45) Date of publication of patent specification: **29.06.88**

(21) Application number: **84100191.0**

(22) Date of filing: **10.01.84**

(51) Int. Cl.⁴: **C 12 N 1/20, C 12 N 9/78**

(54) Method for cultivation of pseudomonas bacteria.

(30) Priority: **10.01.83 JP 1997/83**
**13.10.83 JP 191636/83**

(43) Date of publication of application:
**15.08.84 Bulletin 84/33**

(45) Publication of the grant of the patent:
**29.06.88 Bulletin 88/26**

(84) Designated Contracting States:
**DE FR GB NL**

(56) References cited:
**EP-A-0 093 782**
**AGRICULTURAL AND BIOLOGICAL CHEMISTRY, vol. 46, no. 5, 1982, pages 1165-1174, Agricultural Chemical Society of Japan, Tokyo, JP; Y. ASANO et al.: "Aliphatic nitrile hydratase from Arthrobacter sp. J-1 purification and characterization"**
**AGRICULUTURAL AND BIOLOGICAL CHEMISTRY, vol. 46, no. 5, May 1982, pages 1183-1189, Agricultural Chemical Society of Japan, Tokyo, JP; Y. ASANO et al.: "A new enzymatic method of acrylamide production"**

(73) Proprietor: **NITTO KAGAKU KOGYO KABUSHIKI KAISHA**
**5-1, Marunouchi 1-chome**
**Chiyoda-Ku Tokyo-To (JP)**

(73) Proprietor: **YAMADA, Hideaki**
**19-1 Kinomoto-cho Matsugasaki Sakyo-ku**
**Kyoto-shi Kyoto 606 (JP)**

(72) Inventor: **Yamada, Hideaki**
**19-1, Matsugasaki-Kinomoto-Cho Sakyo-ku**
**Kyoto-shi Kyoto-fu (JP)**
Inventor: **Ryuno, Koitchiro**
**4-13-8, Kishiya Tsurumi-ku**
**Yokohama-shi Kanagawa-ken (JP)**
Inventor: **Enomoto, Kanehiko**
**298-8, Setogaya-cho Hodogaya-ku**
**Yokohama-shi Kanagawa-ken (JP)**
Inventor: **Watanabe, Ichiro**
**2-9-2, Sakuragaoka**
**Yokosuka-shi Kanagawa-ken (JP)**

(74) Representative: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

## Description

Background of the invention

The present invention relates to a method of producing in a high yield cells of *Pseudomonas* bacteria having a high nitrile hydratase activity.

In recent years, the technology of immobilized enzymes or microorganisms has developed rapidly, resulting in increasing attempts to utilize microorganisms and enzymes as they are or in an immobilized state as catalysts for various single or complex chemical reactions.

Nitrile hydratase has been found by Hideaki Yamada, one of the present inventors, et al. as an enzyme capable of hydrating nitriles to produce the corresponding amides. (Reference: Agric. Biol. Chem. *46* 1165(1982)) As one example of the utilization of this enzyme, a method for preparation of acrylamide from acrylonitrile in the presence of bacteria having nitrile hydratase has been proposed. (References: Japanese Patent Laid-Open Pub. No. 86093/1983 (Japanese Patent Appln. No. 184688/1981) and Agric. Biol. Chem. *46* 1183(1982)).

Under these circumstances, a method that can ensure the production of cells of *Pseudomonas* bacteria having a high nitrile hydratase activity in a high yield would be remarkably beneficial.

Summary of the invention

An object of the present invention is to provide a method for production of cells of *Pseudomonas* bacteria having a high nitrile hydratase activity in a high yield by adding α-amino acids to a culture medium in the cultivation of such bacteria.

Thus, a distinguishing feature of the method for cultivation of *Pseudomonas* bacteria having a high nitrile hydratase activity according to this invention is the addition of at least one α-amino acid to a culture medium in the preparation of cells of bacteria having nitrile hydratase activity by cultivating under nitrile hydratase-inducing conditions *Pseudomonas* bacteria capable of producing nitrile hydratase.

We have found that, by adding one or more α-amino acids to the culture medium during the cultivation of *Pseudomonas* bacteria, the nitrile hydratase activity per unit culture fluid increases remarkably. More specifically, for example, the addition of one or more α-amino acids can increase the nitrile hydratase activity per unit culture fluid to a level nearly twice to five times that obtained when the α-amino acid is not added.

This increase in nitrile hydratase activity per unit culture fluid is presumably traceable to the increase in cell concentration (i.e., yield) and cell activity (i.e., quantity of the nitrile hydratase in the cells).

Detailed description of the invention

*Pseudomonas* bacteria

The bacteria used in the present invention are *Pseudomonas* bacteria having nitrile hydratase activity and the capability of hydrating nitriles, particularly acrylonitrile, to produce the corresponding amides, particularly acrylamide. Specific examples of such bacteria are *Pseudomonas chlororaphis,* strain B 23 (FERM BP-187), and *Pseudomonas* sp., strain PS 1 (FERM BP-188), disclosed in Japanese Patent Laid-Open Pub. No. 86093/1983 mentioned above. The principal mycological properties of these bacteria are as follows.

|  |  |  | B 23 | PS 1 |
|---|---|---|---|---|
| (a) |  | Morphology |  |  |
|  | 1 | Shape and size of cell | bacillus 0.8—1.1×1.6—2.7 μm | bacillus 0.8—1.1×1.3—1.9 μm |
|  | 2 | Polymorphism | none | none |
|  | 3 | Motility | motile one to three polar flagella | motile with polar flagella |
|  | 4 | Formation of spores | none | none |
|  | 5 | Gram staining | − | − |
|  | 6 | Acid-fast property | − | − |
| (b) |  | Growth on various culture media |  |  |
|  | 1 | Bouillon-agar plate culture | spherical, convex, glossy, translucent and yellow | smooth, homogeneous, glossy, and mucoidal |
|  | 2 | Bouillon-agar slant culture | small colony formed | smooth, glossy, translucent, and yellow |
|  | 3 | Bouillon liquid culture | precipitated |  |
|  | 4 | Bouillon-gelatin stab culture | liquified (+) | − |
|  | 5 | Litmus-milk | acidic: peptonized, not coagulated | alkaline: peptonized, not coagulated |
| (c) |  | Physiological properties |  |  |
|  | 1 | Reduction of nitrate | + | − |
|  | 2 | Denitrification | + | − |
|  | 3 | MR test | − | − |
|  | 4 | VP test | − | − |
|  | 5 | Formation of indole | − | − |
|  | 6 | Formation of hydrogen sulfide | − | − |
|  | 7 | Hydrolysis of starch | − | − |
|  | 8 | Utilization of citric acid | Simon's culture: + | Simon's culture: + |
|  | 9 | Utilization of inorganic nitrogen source | ammonium salt: + | ammonium salt: + |
|  | 10 | Formation of pigments | King-A culture: − King-B culture: + green (water-soluble) | King-A culture: − King-B culture: + green (water-soluble) |
|  | 11 | Urease | − | − |
|  | 12 | Oxidase | + | + |

|  |  |  | B 23 | | PS 1 | |
|---|---|---|---|---|---|---|
| (c) | Physiological properties |  |  | | | |
| 13 | Catalase |  | + | | + | |
| 14 | Growth range |  | pH: 6.0—9.9 temperature: 5—36.5°C | | | |
| 15 | Behavior toward oxygen |  | aerobic | | aerobic | |
| 16 | O—F Test |  | oxidized | | oxidized | |
| 17 | Formation of acid & gas from saccharide | | Formation of acid | Formation of gas | Formation of acid | Formation of gas |
|  |  | D-glucose | + | – | + | – |
|  |  | D-mannose | + | – | + | – |
|  |  | D-fructose | – | – | – | – |
|  |  | D-galactose | + | – | + | – |
|  |  | maltose | – | – | – | – |
|  |  | sucrose | – | – | – | – |
|  |  | lactose | – | – | – | – |
|  |  | trehalose |  |  | – | – |
|  |  | D-mannitol | – | – | – | – |
|  |  | glycerol | – | – | – | – |
|  |  | starch | – | – | – | – |
| 18 | Nutritive requirements |  | none | | none | |
| 19 | Other properties |  | See remarks | | | |

Remarks:

|  |  |
|---|---|
| Aminopeptidase | + |
| Formation of levan from saccharose | + |
| Formation of poly-β-hydroxybutyrate | – |
| GC content | 64.6% |

Enzymatic activity improving agent

In the present invention, one or more α-amino acids are used as enzymatic activity improving agents. These α-amino acids can be used singly or in the form of a mixture of two or more members.

The α-amino acids used in the present invention may be of the L-type, D-type or mixed L- and D-types, and can be used singly or in the form of a mixture of two or more members as has been set forth above. L-typed α-amino acids may be preferred from the point of view of effectiveness while mixtures of D- and L-typed α-amino acids may be preferred from the point of view of easy availability.

Suitable α-amino acids are those which are known as natural protein constituents. Specific examples of such α-amino acids are those whose effectiveness is indicated in Examples 1 through 7 set forth hereinlater. Especially preferred among these α-amino acids are cysteine, cystin, alanine, valine, methionine, aspartic acid, phenylalanine, proline, and glutamic acid. Of these, cysteine, cystin, methionine, aspartic acid, and glutamic acid are advantageous because of their relatively stable activity.

Cultivation-practice of the present invention

A preferred embodiment of this invention will be described below.

At least one α-amino acid is added at one time or sequentially to a culture medium containing: carbon sources such as glucose, fructose, sucrose, dextrins, glycerol, ethanol, and succinic acid; nitrogen sources such as ammonia, ammonium sulfate, ammonium chloride, ammonium nitrate, and urea; organic nutriment sources such as yeast extract, meat extract, malt extract, casein hydrolyzate, and peptone; inorganic salts such as phosphates; magnesium, potassium, and iron and like metals in trace amounts; and other substances at a concentration of 0.1 to 10 g/litre, preferably 0.5 to 6.0 g/litre. The term "sequentially" as used herein is intended to mean both "continuously" and "intermittently".

This culture medium is inoculated with *Pseudomonas* bacteria having nitrile hydratase activity, and cultivation is carried out under aerobic conditions while an enzyme inducing agent is added to induce nitrile hydratase. Examples of the enzyme inducing agent are propionitrile, isobutyronitrile, propionamide,

4

and isobutyramide (Japanese Patent Appln. No. 199750/1982), and acrylamide, methacrylamide, crotonamide, and n-butyramide (Japanese Patent Appln. No. 191637/1983). These enzyme inducing agents are effective when added during the cultivation of bacteria at a concentration ordinarily of lower than 15 g/litre, preferably of 10 g/litre or lower. The pH of the culture medium is of the order of 6 to 9, preferably of the order of 7 to 8, while the cultivation temperature is of the order of 20 to 37°C, preferably of the order of 25 to 30°C, and the cultivation time is about 1 to 3 days.

Experimental Examples

In each of Examples 1 through 4 of the following experimental examples, 1 ml of a culture fluid was added to 9 ml of a phosphate buffer solution (pH 7.5) containing 2.8% by weight of acrylonitrile, and the resulting solution was caused to react at 10°C for 10 to 60 minutes. The quantity of acrylamide obtained was measured by means of gas chromatography, and the hydratase activity of the bacteria exhibited in the hydration of acrylonitrile was determined on the basis of the data thus obtained, the capability of producing 1 μmole of acrylamide per ml of a culture fluid per minute being designated as 1 unit.

In Examples 5, 6 and 7, the hydratase activity of the bacteria exhibited in the hydration of acrylonitrile was determined in accordance with a procedure which will be described separately in Example 5.

Example 1

To five separate but identical lots of a culture medium, each comprising 10 g/litre of sucrose, 2 g/litre of $K_2HPO_4$, 0.5 g/litre of $MgSO_4 \cdot 7H_2O$, 1 g/litre of NaCl, and 10 mg/litre of $FeSO_4 \cdot 7H_2O$ was added L-cysteine at respectively different concentrations ranging from 0.1 to 5.0 g/litre. The pH of each culture medium was adjusted to 7.2, and 100 ml of each resulting culture medium was sterilized in a 500-ml Erlenmeyer flask.

After cooling, 0.4 g of isobutyronitrile was added to each sterilized culture medium which was then inoculated with 0.5 ml of a culture fluid obtained by precultivating *Pseudomonas chlororaphis,* strain B 23 (FERM BP-187), in a culture medium of the above composition containing no L-cysteine, and shaking cultivation was carried out aerobically at 25°C for 2 days.

For comparison purposes, cultivation was carried out similarly without addition of L-cysteine.

The cell concentration of each of the culture fluids and the nitrile hydratase activity thereof exhibited in the hydration of acrylonitrile were measured. These results obtained are shown in Table 1.

TABLE 1

| Quantity of L-cysteine added (g/litre) | Cell concentration (g/litre) | Nitrile hydratase activity (unit) |
|---|---|---|
| 0 Comparison Example | 1.39 | 20.3 |
| 0.1 | 1.37 | 27.1 |
| 0.5 | 1.40 | 33.3 |
| 1.0 | 1.50 | 42.2 |
| 2.0 | 1.80 | 56.3 |
| 5.0 | 1.79 | 39.9 |

Example 2

To three separate but identical lots of culture medium, each comprising 10 g/litre of sucrose, 2 g/litre of $K_2HPO_4$, 0.5 g/litre of $MgSO_4 \cdot 7H_2O$, 1 g/litre of NaCl, and 10 mg/litre of $FeSO_4 \cdot 7H_2O$ were added D-cysteine, D,L-cysteine, and L-cystin respectively in three instances at a concentration of 1.0 g/litre. The pH of each culture medium was adjusted to 7.2, and 100 ml of each resulting culture medium was sterilized in a 500-ml Erlenmeyer flask.

After cooling, 0.4 g of isobutyronitrile was added to each sterilized culture medium which was then inoculated with 0.5 ml of a culture fluid obtained by precultivating *Pseudomonas chlororaphis,* strain B 23 (FERM BP-187), in a culture medium of the above composition containing no cysteine or cystin, and shaking cultivation was carried out aerobically at 25°C for 2 days.

For comparison purposes, cultivation was carried out similarly without addition of cysteine or cystin.

The cell concentration of each of the culture fluids and the nitrile hydratase activity thereof exhibited in the hydration of acrylonitrile were measured. The results obtained are set forth in Table 2.

TABLE 2

| Species of additive | Quantity (g/litre) | Cell concentration (g/litre) | Nitrile hydratase activity (unit) |
|---|---|---|---|
| No additive (Comparison Example) | 0 | 1.41 | 21.2 |
| D-Cysteine | 1.0 | 1.39 | 34.0 |
| D,L-Cysteine | 1.0 | 1.40 | 40.7 |
| L-Cystin | 1.0 | 1.45 | 38.3 |

Example 3

To two separate but identical lots of a culture medium, each comprising 10 g/litre of glycerol, 2 g/litre of $K_2HPO_4$, 0.5 g/litre of $MgSO_4 \cdot 7H_2O$, 1 g/litre of NaCl, and 10 mg/litre of $FeSO_4 \cdot 7H_2O$ were added L-cysteine and L-cystin, respectively, in two instances at a concentration of 1.0 g/litre. The pH of each culture medium was adjusted to 7.2, and 100 ml of each resulting culture medium was sterilized in a 500-ml Erlenmeyer flask.

After cooling, 0.8 g of propionitrile was added to each sterilized culture medium, which was then inoculated with 0.5 ml of a culture fluid obtained by precultivating *Pseudomonas* sp., strain PS 1 (FERM BP-188), in a culture medium of the above composition containing no cysteine or cystin, and shaking cultivation was carried out aerobically at 25°C for 2 days.

For comparison purposes, cultivation was carried out under similar conditions except that neither cysteine nor cystin was added.

The cell concentration of each of the culture fluids and the nitrile hydratase activity thereof exhibited in the hydration of acrylonitrile were measured. The results obtained were as shown in Table 3.

TABLE 3

| Species of additive | Quantity (g/litre) | Cell concentration (g/litre) | Nitrile hydratase activity (unit) |
|---|---|---|---|
| No additive (Comparison Example) | 0 | 3.21 | 15.8 |
| L-Cysteine | 1.0 | 3.38 | 30.5 |
| L-Cystin | 1.0 | 3.31 | 27.6 |

Example 4

To two each separate but identical lots of a culture medium, each comprising 10 g/litre of sucrose, 2 g/litre of $K_2HPO_4$, 0.5 g/litre of $MgSO_4 \cdot 7H_2O$, 1 g/litre of NaCl, 10 mg/litre of $FeSO_4 \cdot 7H_2O$, and 2 g/litre of yeast extract was added L-cysteine at a concentration of 1 and 2 g/litre, respectively. The pH of each culture medium was adjusted to 7.2, and 100 ml of each resultant culture medium was sterilized in a 500-ml Erlenmeyer flask.

After cooling, 0.4 g of isobutyronitrile was added to each sterilized culture medium, which was then inoculated with 0.5 ml of a culture fluid obtained by precultivating *Pseudomonas chlororaphis,* strain B 23 (FERM BP-187), in a culture medium of the above composition containing no L-cysteine, and cultivation was carried out under aerobic conditions at 25°C for 2 days.

For comparison purposes, cultivation was carried out similarly without addition of L-cysteine. The cell concentration of each of the culture fluids and nitrile hydratase activity thereof were measured, whereupon the results shown in Table 4 were obtained.

TABLE 4

| Quantity of L-cysteine added (g/litre) | Cell concentration (g/litre) | Nitrile hydratase activity (unit) |
|---|---|---|
| 0 (Comparison Example) | 3.32 | 36.5 |
| 1.0 | 3.53 | 73.1 |
| 2.0 | 4.05 | 105.7 |

Example 5

1. Cultivation of bacteria

2 ml of a seed culture fluid obtained from *Pseudomonas chlororaphis,* strain B 23 (FERM BP-187), grown under the following precultivation conditions was cultivated under the following cultivation conditions to determine the acrylamide-producing activity of the bacteria.

(1) Precultivation conditions

MY Culture Medium (pH 7.6):

| peptone | 5 g/litre |
|---|---|
| yeast extract | 3 g/litre |
| malt extract | 3 g/litre |
| glucose | 5 g/litre |

Cultivation temperature: 28°C
Cultivation time: 12 hours
A 500-ml (net capacity: 100 ml) Sakaguchi-flask was used.

(2) Cultivation Conditions

Culture medium (pH 7.6):

| sucrose | 10 g/litre |
|---|---|
| $KH_2PO_4$ | 0.5 g/litre |
| $K_2HPO_4$ | 0.5 g/litre |
| $MgSO_4 \cdot 7H_2O$ | 20 mg/litre |
| α-amino acid | 2 g/litre |
| isobutyronitrile | 5 ml/litre |

Cultivation temperature: 25°C
A 500-ml (net capacity: 100 ml) Sakaguchi-flask was used.

2. Measurement of nitrile hydratase activity

1 ml of a culture fluid was admixed with 4 ml of a 1/10 M phosphate buffer solution (pH 7.0), and 5 ml of a 1/10 M phosphate buffer solution (pH 7.0) containing 5.0% by weight of acrylonitrile was added thereto. The resulting solution was caused to react at 10°C for 10 minutes, and the bacterium cells therein were separated by filtration. The nitrile hydratase activity of the cells exhibited in the hydration of acrylonitrile to produce acrylamide was determined by measuring the quantity of the acrylamide (AA) thus produced by means of gas chromatography.

The activity was determined for the specific activity (SA) and the total activity (TA) as defined below.

SA: μmole AA/mg-cells/min.
TA: μmole AA/ml-culture medium/min.

The results obtained are summarized in Table 5 in which the activity is shown by the maximum activity value marked.

TABLE 5

| Species of Amino acid[*1] | Cultivation time (hr) | Cell concentration[*2] (g/litre) | Enzymatic activity | |
|---|---|---|---|---|
| | | | SA | TA |
| DL-Ala | 42 | 3.04 | 23.27 | 70.62 |
| L-Val | 48 | 3.07 | 23.39 | 71.81 |
| L-Met | 36 | 2.04 | 35.53 | 72.48 |
| L-Asp | 36 | 4.48 | 16.88 | 75.28 |
| L-Lys | 48 | 2.28 | 24.55 | 55.97 |
| L-Phe | 42 | 2.90 | 28.19 | 81.85 |
| L-Pro | 36 | 3.18 | 25.72 | 81.79 |
| L-Trp | 42 | 2.16 | 23.26 | 50.24 |
| L-Leu | 39 | 3.16 | 15.84 | 50.05 |
| L-Ile | 32 | 3.28 | 14.10 | 46.25 |
| L-Glu | 26 | 3.36 | 23.05 | 77.45 |
| L-Arg | 26 | 3.54 | 17.90 | 63.37 |
| L-His | 32 | 3.04 | 17.03 | 51.77 |
| L-Tyr | 32 | 2.11 | 23.06 | 48.66 |
| L-Cys | 32 | 2.44 | 26.08 | 63.64 |
| L-CysS | 30 | 2.14 | 34.47 | 73.76 |
| —[*3] | 42 | 1.80 | 22.22 | 40.00 |

[*1]　Ala: alanine　　Leu: leucine
Val: valine　　Ile: isoleucine
Met: methionine　Glu: glutamic acid
Asp: aspartic acid　Arg: arginine
Lys: lysine　　His: histidine
Phe: phenylalanine　Tyr: tyrosine
Pro: proline　　Cys: cysteine
Trp: tryptophan　CysS: cystin

[*2]　on a dry cell basis
[*3]　amino acid not added

Example 6

Cultivation was carried out under the same conditions as in Example 5 with combinations of α-amino acids which had been found to be especially effective in Example 5 and cysteine respectively at a concentration of 2 g/litre. The activity is shown by the maximum activity value marked.

TABLE 6

| Species of amino acid | Cultivation time (hr) | Cell concentration (g/litre) | Enzymatic activity | |
|---|---|---|---|---|
| | | | SA | TA |
| DL-Ala | 31 | 3.58 | 27.35 | 97.91 |
| L-Val | 31 | 3.34 | 27.39 | 91.48 |
| L-Met | 37 | 3.52 | 24.64 | 86.73 |
| L-Asp | 31 | 5.48 | 16.94 | 92.83 |
| L-Phe | 31 | 3.10 | 30.71 | 95.20 |
| L-Pro | 31 | 3.58 | 29.09 | 104.14 |
| L-Glu | 31 | 4.05 | 25.53 | 103.40 |
| —* | 32 | 2.44 | 26.08 | 63.64 |

* L-Cysteine alone

Example 7

Cultivation was carried out similarly as in Example 6 by the use of some α-amino acids with the Cys-Glu combination which had been found effective in Example 6, the concentration of the cysteine, glutamic acid and α-amino acid each being 2 g/litre. The activity is shown by the maximum activity value marked.

TABLE 7

| Species of amino acid | Cultivation time (hr) | Cell concentration (g/litre) | Enzymatic activity | |
|---|---|---|---|---|
| | | | SA | TA |
| L-Phe | 33 | 5.42 | 25.88 | 140.3 |
| L-Pro | 31 | 6.08 | 25.88 | 157.4 |
| L-Asp | 33 | 5.84 | 20.65 | 120.6 |
| DL-Ala | 33 | 6.02 | 22.51 | 135.5 |
| —* | 31 | 4.05 | 25.53 | 103.4 |

* L-cysteine+L-glutamic acid alone.

**Claims**

1. A method for cultivation of *Pseudomonas* bacteria, characterized in that at least one α-amino acid is added to a culture medium in the preparation of cells of bacteria having nitrile hydratase activity by cultivating under nitrile hydratase-inducing conditions *Pseudomonas* bacteria capable of producing nitrile hydratase.

2. A method as claimed in claim 1, characterized in that the concentration of the α-amino acid in the culture medium is in the range of from 0.1 to 10 g/litre.

3. A method as claimed in claim 1, characterized in that the *Pseudomonas* bacteria capable of producing nitrile hydratase is *Pseudomonas chlororaphis,* strain B 23 (FERM BP-187), or *Pseudomonas* sp., strain PS 1 (FERM BP-188).

4. A method as claimed in claim 1, characterized in that the α-amino acid is selected from the group consisting of cysteine, cystin, alanine, valine, methionine, aspartic acid, lysine, phenylalanine, proline, tryptophan, leucine, isoleucine, glutamic acid, arginine, histidine and tyrosine.

5. A method as claimed in claim 1, characterized in that the α-amino acid is selected from the group

## 0 115 781

consisting of cysteine, cystin, alanine, valine, methionine, aspartic acid, phenylalanine, proline and glutamic acid.

6. A method as claimed in claim 1, characterized in that the α-amino acid is selected from the group consisting of cysteine, cystin, methionine, aspartic acid and glutamic acid.

**Patentansprüche**

1. Verfahren zur Züchtung von Pseudomonas-Bakterien, dadurch gekennzeichnet, daß mindestens eine α-Aminosäure zu dem Kulturmedium bei der Herstellung der Bakterienzellen mit Nitrilhydratase-Aktivität zugegeben wird, wobei Pseudomonas-Bakterien, welche Nitrilhydratase bilden können, unter solchen Bedingungen gezüchtet werden, daß die Bildung von Nitrilhydratase induziert wird.

2. Verfahren nach Anspruch 1, dadurch gekennzeichnet, daß die Konzentration der α-Aminosäure in dem Kulturmedium im Bereich von 0,1 bis 10 g/l liegt.

3. Verfahren nach Anspruch 1, dadurch gekennzeichnet, daß das Pseudomonas-Bakterien, welche Nitrilhydratase bilden können, Pseudomonas chlororaphis Stamm B 23 (FERM BP-187) oder Pseudomonas sp. Stamm PS 1 (FERM BP-188) verwendet werden.

4. Verfahren nach Anspruch 1, dadurch gekennzeichnet, daß die α-Aminosäure aus der Gruppe aus gewählt wird Cystein, Cystin, Alanin, Valin, Methionin, Asparaginsäure, Lysin, Phenylalanin, Prolin, Tryptophan, Leucin, Isoleucin, Glutaminsäure, Arginin, Histidin und Tyrosin.

5. Verfahren nach Anspruch 1, dadurch gekennzeichnet, daß die α-Aminosäure aus der Gruppe ausgewählt wird Cystein, Cystin, Alanin, Valin, Methionin, Asparaginsäure, Phenylalanin, Prolin und Glutaminsäure.

6. Verfahren nach Anspruch 1, dadurch gekennzeichnet, daß die α-Aminosäure aus der Gruppe ausgewählt wird Cystein, Cystin, Methionin, Asparaginsäure und Glutaminsäure.

**Revendications**

1. Une méthode de culture de bactéries *Pseudomonas* caractérisée en ce que l'on ajoute un ou plusieurs α-aminoacides à un milieu de culture dans la formation de cellules de bactéries ayant une activité de nitrile hydratase par culture de bactéries *Pseudomonas* pouvant produire la nitrile hydratase dans des conditions qui induisent la formation de cette enzyme.

2. Une méthode selon la revendication 1 caractérisée en ce que la teneur du milieu de culture en α-aminoacide est de 0,1 à 10 g/litre.

3. Une méthode selon la revendication 1 caractérisée en ce que la bactérie *Pseudomonas* produisant la nitrile hydratase est la bactérie *Pseudomonas chlororaphis* souche B 23 (FERM BP-187), ou *Pseudomonas* sp., souche PS 1 (FERM BP-188).

4. Une méthode selon la revendication 1 caractérisée en ce que l'α-aminoacide est choisi parmi la cystéine, la cystine, l'analine, la valine, la méthionine, l'acide aspartique, la lysine, la phénylalanine, le tryptophane, la leucine, l'isoleucine, l'acide glutamique, l'arginine, l'histidine et la tyrosine.

5. Une méthode selon la revendication 1 caractérisée en ce que l'α-aminoacide est choisi parmi la cystéine, la cystine, l'alanine, la valine, la méthionine, l'acide aspartique, la phénylalanine, la proline et l'acide glutamique.

6. Une méthode selon la revendication 1 caractérisée en ce que l'α-aminoacide est choisi parmi la cystéine, la cystine, la méthionine, l'acide aspartique et l'acide glutamique.

10